# EUROPEAN PATENT SPECIFICATION

(11) **EP 2 505 836 B1**
(45) Date of publication and mention of the grant of the patent: **29.07.2020**
(21) Application number: 10833180.2
(22) Date of filing: 22.11.2010
(51) Int. Cl.: F04B 11/00, A61M 1/14, F04B 3/00, F04B 9/02, F04B 23/06, A61M 1/16, F04B 43/02

(54) **RECIPROCATION PUMP AND DIALYSIS DEVICE COMPRISING SAME**
HUBKOLBENPUMPE UND DIALYSEVORRICHTUNG DAMIT
POMPE ALTERNATIVE ET DISPOSITIF DE DIALYSE COMPRENANT CETTE POMPE

(30) Priority: 24.11.2009 JP 2009266394
(43) Date of publication of application: 03.10.2012
(73) Proprietor: Nikkiso Company Limited, Tokyo 150-8677 (JP)
(72) Inventor: SUZUKI, Hiroaki, Makinohara-shi Shizuoka 421-0496 (JP)
(74) Representative: von Hirschhausen, Helge
(86) International application number: PCT/JP2010/070827
(87) International publication number: WO 2011/065334

(56) References cited:
- JP-A- 50 024 675
- JP-A- 2003 003 966
- JP-A- 2003 284 772
- JP-A- 2003 284 772
- JP-U- 50 004 304
- US-A- 4 600 365
- US-A- 4 676 905
- US-A- 4 761 118
- US-A- 4 857 199
- US-A1- 2004 164 013
- US-A1- 2005 254 971

## Description

### Field of the invention

The present invention relates to a reciprocation pump for supplying a liquid from the source to an objective apparatus with the supply liquid-side pumping chamber and discharging to the outside a waste liquid from the objective apparatus with the waste liquid-side pumping chamber, and to a dialysis apparatus equipped with the reciprocation pump.

### Description of Background Art

In general, a blood purification apparatus such as a dialyzer is used in hemodialysis treatment and a dialysate supplying line for supplying the dialysate as well as a dialysate discharging line for discharging the waste dialysate are connected to the blood purification apparatus. These dialysate supplying line and dialysate discharging line are extended from a dialysis apparatus main body and connected to the blood purification apparatus to supply the dialysate to the blood purification apparatus and to discharge the waste dialysate from the blood purification apparatus to the outside.

The reciprocation pump (duplex pump) is connected to the dialysis apparatus main body across the dialysate supplying line and the dialysate discharging line. As shown for example in Patent Document 1, the reciprocation pump comprises a casing for reciprocatably accommodating a plunger, a supply liquid-side pumping chamber and a waste liquid-side pumping chamber formed by a plunger within the casing, and a motor M for reciprocating the plunger.

The supply liquid-side pumping chamber and the waste liquid-side pumping chamber are provided with a suction valve and a discharge valve formed as check valves and the reciprocation pump is so structured that the supply liquid-side pumping chamber supplies dialysate to a blood purification apparatus and the waste liquid-side pumping chamber discharges waste dialysate from the blood purification apparatus to the outside thereof. This is performed by the reciprocating motion of the plunger driven by a motor.

In US 4,857,199 a system for pumping a first and a second liquid in an artificial kidney in substantially equal quantities at a substantially constant flow is disclosed including a main pump having complementary main chambers, and first and second auxiliary pumps each having complementary auxiliary chambers. The pumps are reciprocated in unison and each main chamber is connected to an auxiliary chamber which functions as a dead-end reservoir for the respective main chamber.

For reducing the pulsation arising from the compressibility of a liquid to be delivered, US 4,600,365 teaches two series-connected cylinders which pistons are controlled through cams which are driven jointly at a constant velocity. The cams are so formed that the transition phase preceding the delivery phase (in the direction of delivery) of the after-cylinder is longer than the transition phase preceding the delivery phase (delivery direction) of the forward cylinder.

Japanese Laid-open Patent Publication No. 2003-284772 discloses a system comprising a pump to supply and discharge simultaneously fluid to and from a dialyzer. This document does not disclose a supply liquid-side sub-pumping chamber and a waste liquid-side sup-pumping chamber.

### Disclosure of the Invention

### Problems to be solved by the Invention

However, in such a reciprocation pump of the prior art described above, it is necessary to increase the volume of a mixing chamber arranged at a dialysate conditioning section for mixing clean water for dialysis and stock dialysate due to the pulsation caused by reciprocating motion of the plunger. In addition, it is afraid that the pulsation caused by reciprocating motion of the plunger would be excessively large and thus various undesirable problems would be caused when trying to supply a large amount of dialysate to the blood purification apparatus. That is, increase of the flow rate of the dialysate would cause increase of the internal pressure within dialysate pipes due to the flow resistance of them and thus it is necessary that the piping elements in various piping sections should be adapted to high pressure when trying to supply a large amount of dialysate to the blood purification apparatus. These problems will be similarly encountered in reciprocation pumps for supplying liquids to objective apparatus in other fields than dialysis.

It is, therefore, an object of the present invention to provide a reciprocation pump which can reduce the pulsation of the liquid and waste liquid caused by the reciprocating motion of the reciprocation pump and also to provide a dialysis apparatus equipped with such a reciprocation pump.

### Means for solving problems

For achieving the object mentioned above, there is provided, according to the present invention of claim 1, a system comprising an objective apparatus and a reciprocation pump, the reciprocation pump comprising a supply liquid-side pumping chamber configured to supply a liquid from a source to an objective apparatus; a waste liquid-side pumping chamber configured to discharge to the outside a waste liquid discharged from the objective apparatus; a reciprocation means being able to reciprocate between the supply liquid-side pumping chamber and the waste liquid-side pumping chamber, the reciprocating motion of the reciprocation means performing the suction and discharge of the liquid to and from the supply liquid-side pumping chamber as well as the suction and discharge of the waste liquid to and from the waste liquid-side pumping chamber; and a driving source for driving the reciprocation means; the reciprocation pump adapted to supply the liquid from the source to the objective apparatus with the supply liquid-side pumping chamber and to discharge to the outside the waste liquid from the objective apparatus with the waste liquid-side pumping chamber; the reciprocation pump further comprises a supply liquid-side sub-pumping chamber for containing and sending out liquid adapted to be communicated with the supply liquid-side pumping chamber and interlocked with the reciprocation means to vary its volume reversely to that of the volume variation of the supply liquid-side pumping chamber; and a waste liquid-side sub-pumping chamber for containing and sending out liquid adapted to be communicated with the waste liquid-side pumping chamber and interlocked with the reciprocation means to vary its volume reversely to that of the volume variation of the waste liquid-side pumping chamber; and that the reciprocating motion of the reciprocation means enables the liquid to be supplied to the objective apparatus through the supply liquid-side pumping chamber and the supply liquid-side sub-pumping chamber as well as enables the waste liquid from the objective apparatus to be discharged to the outside through the waste liquid-side pumping chamber and the waste liquid-side sub-pumping chamber. According to the invention, the supply liquid-side pumping chamber comprises a supply liquid-side inlet port and a supply liquid-side outlet port and the supply liquid-side sub-pumping chamber comprises connection ports, and the waste liquid-side pumping chamber comprises a waste liquid-side inlet port and a waste liquid-side outlet port and the waste liquid-side sub-pumping chamber comprises connection ports.

The present invention of claim 2 is a system of claim 1 characterized in that the reciprocation pump further comprises a rod extending through the supply liquid-side sub-pumping chamber, the supply liquidside pumping chamber, the waste liquid-side pumping chamber and the waste liquid-side sub-pumping chamber and being connected to the reciprocation means and the driving source to transmit a driving force of the driving source to the reciprocation means; and diaphragms respectively mounted on the distal end and the proximal end of the rod to form the supply liquid-side sub-pumping chamber and the waste liquid-side sub-pumping chamber respectively.

The present invention of claim 3 is a system of claim 1 or 2 characterized in that the reciprocating pump is configured to supply liquid from the source to the objective apparatus through the supply liquid-side pumping chamber via the supply liquid-side sub-pumping chamber and to discharge liquid from the objective apparatus to the outside through the waste liquid-side sub-pumping chamber via the waste liquid-side pumping chamber.

The present invention of claim 4 is a system of claim 1 or 2, wherein the reciprocating pump is configured to supply liquid from the source to the objective apparatus through the supply liquid-side sub-pumping chamber via the supply liquid-side pumping chamber and to discharge liquid from the objective apparatus to the outside through the waste liquid-side pumping chamber via the waste liquid-side sub-pumping chamber.

The present invention of claim 5 is a system of claim 1 or 2, wherein the reciprocating pump is configured to supply liquid from the source to the objective apparatus through the supply liquid-side pumping chamber via the supply liquid-side sub-pumping chamber and to discharge liquid from the objective apparatus to the outside through the waste liquid-side pumping chamber via the waste liquid-side sub-pumping chamber.

The present invention of claim 6 is a system of any one of claims 2∼5 characterized in that the reciprocation means comprises a plunger formed on the rod to form the supply liquid-side pumping chamber and the waste liquid-side pumping chamber.

The present invention of claim 7 is a system of any one of claims 2∼5 characterized in that the reciprocation means comprises a diaphragm mounted on the rod to form the supply liquid-side pumping chamber and the waste liquid-side pumping chamber.

The present invention of claim 8 is a system of any one of claims 1∼7 characterized in that the objective apparatus is a blood purification apparatus and the liquid is dialysate.

The present invention of claim 9 is a dialysis apparatus characterized in that it is equipped with the system of claim 8.

### Effects of the invention

According to the present invention of claim 1, since the reciprocation pump is provided with the supply liquid-side sub-pumping chamber and the waste liquid-side sub-pumping chamber in addition to the supply liquid-side pumping chamber and the waste liquid-side pumping chamber and structured so that the reciprocating motion of the reciprocation means enables the liquid to be supplied to the objective apparatus through the supply liquid-side pumping chamber and the supply liquid-side sub-pumping chamber as well as enables the waste liquid from the objective apparatus to be discharged to the outside through the waste liquid-side pumping chamber and the waste liquid-side sub-pumping chamber, it is possible to reduce the pulsation of the liquid and waste liquid caused by the reciprocating motion of the reciprocation pump.

According to the present invention of claim 2, since the reciprocation pump further comprises a rod extending through the supply liquid-side sub-pumping chamber, the supply liquid-side pumping chamber, the waste liquid-side pumping chamber and the waste liquid-side sub-pumping chamber and being connected to the reciprocation means and the driving source to transmit a driving force of the driving source to the reciprocation means; and diaphragms respectively mounted on the distal end and the proximal end of the rod to form the supply liquid-side sub-pumping chamber and the waste liquid-side sub-pumping chamber respectively, it is possible to construct the supply liquid-side sub-pumping chamber interlocked with the reciprocation means to vary its volume reversely to that of the volume variation of the supply liquid-side pumping chamber and the waste liquid-side sub-pumping chamber interlocked with the reciprocation means to vary its volume reversely to that of the volume variation of the waste liquid-side pumping chamber. In addition, it enables to eliminate any separate sealing means such as shaft seals at mounting portions of the diaphragms.

According to the present invention of claim 6, since the reciprocation means comprises a plunger formed on the rod to form the supply liquid-side pumping chamber and the waste liquid-side pumping chamber, it is possible to use a conventional plunger and thus to reduce the manufacturing cost.

According to the present invention of claim 7, since the reciprocation means comprises a diaphragm mounted on the rod to form the supply liquid-side pumping chamber and the waste liquid-side pumping chamber, it is possible to eliminate any sealing means for forming the supply liquid-side pumping chamber and the waste liquid-side pumping chamber in a sealed manner as compared with a case using plunger for this purpose.

According to the present invention of claim 8, since the objective apparatus is a blood purification apparatus and the liquid is dialysate, it is possible to reduce the pulsation of the liquid and waste liquid caused by the reciprocating motion of the reciprocation pump.

According to the present invention of claim 9, it is possible to provide a dialysis apparatus having superior effects of the system defined in claim 8.

### Brief Description of the Drawings

[Fig.1] A longitudinal-section view of a reciprocation pump of a preferable embodiment of the present invention;

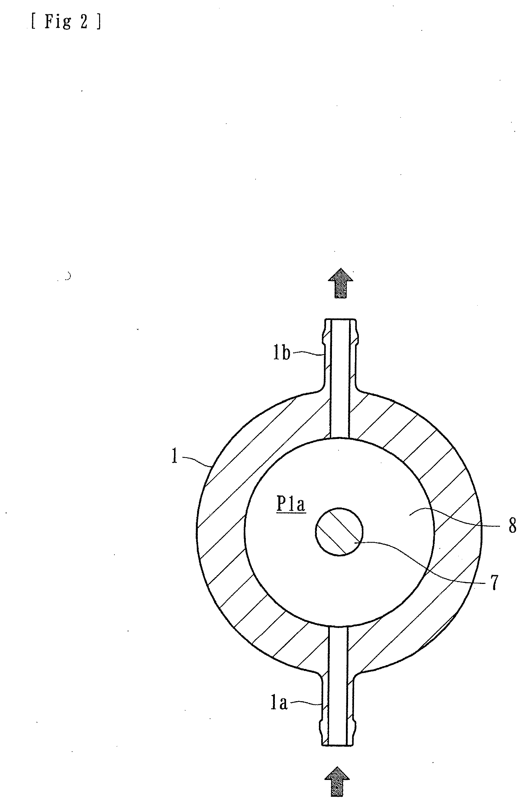

[Fig.2] A cross-sectional view taken along a line II-II of Fig. 1;

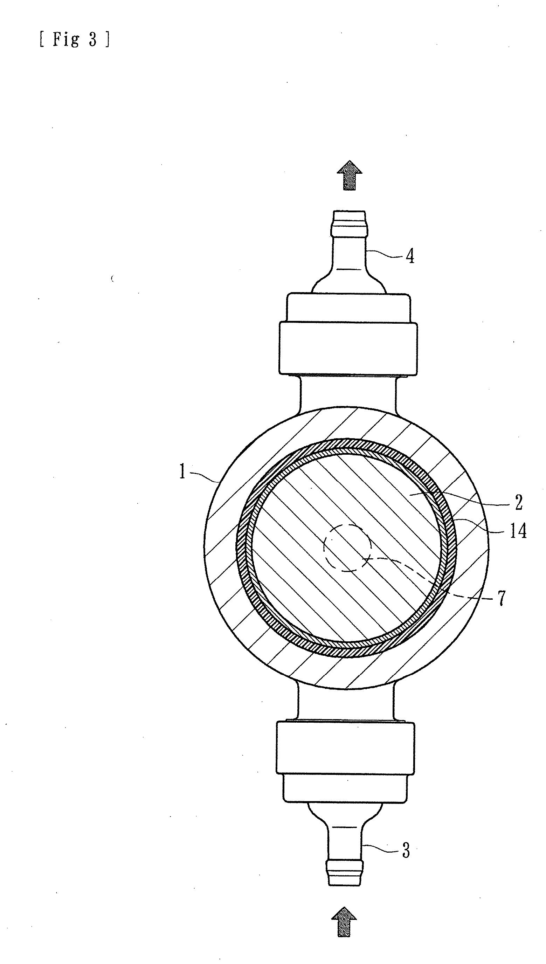

[Fig.3] A cross-sectional view taken along a line III-III of Fig. 1;
[Fig.4] A schematic view showing a connection (first condition of connection) between the reciprocation pump and a blood purification apparatus;
[Fig.5] A graph showing the pulsation under the first condition of connection of the reciprocation pump of Fig. 1;
[Fig.6] A schematic view showing a connection (second condition of connection) between the reciprocation pump and the blood purification apparatus;
[Fig.7] A schematic view showing a connection (third condition of connection) between the reciprocation pump and the blood purification apparatus;
[Fig.8] A graph showing the pulsation under the third condition of connection of the reciprocation pump of Fig. 1; and
[Fig.9] A longitudinal-section view of a reciprocation pump of another embodiment of the present invention;

### A Preferable mode for carrying out the Invention

The preferable embodiments of the present invention will be described with reference to the accompanied drawings.

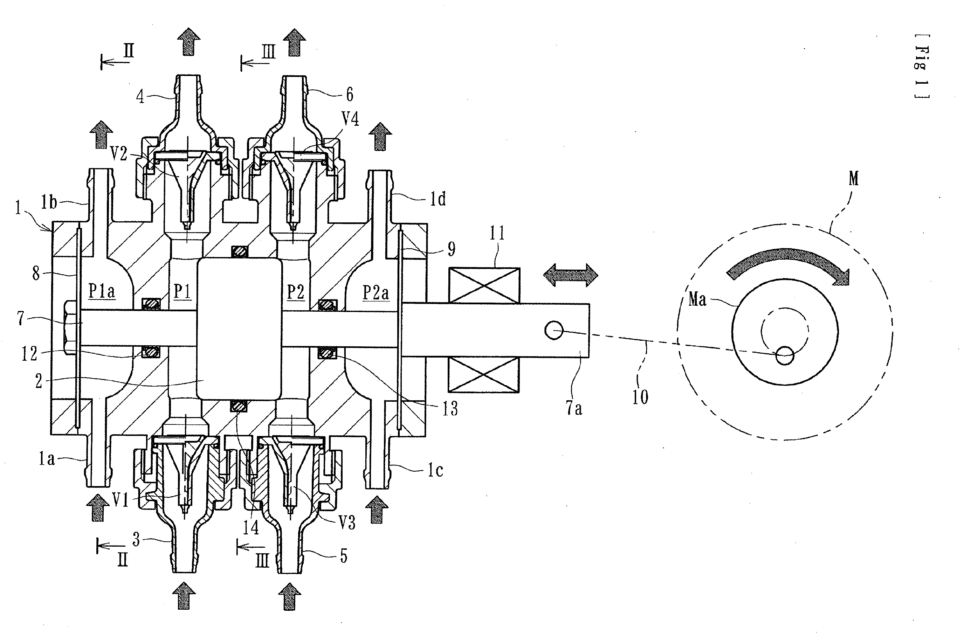

The reciprocation pump of one preferable embodiment of the present invention can be applied to the hemodialysis apparatus and comprises a so-called duplex pump for supplying a liquid to an objective apparatus with a supply liquid-side pumping chamber and discharging a waste liquid from the objective apparatus to a waste liquid-side pumping chamber. As shown in Figs. 1∼3, the reciprocation pump of the present invention mainly comprises a casing 1 in which a supply liquid-side pumping chamber P1 and a waste liquid-side pumping chamber P2 are formed, a plunger 2 acting as a reciprocation means, a supply liquid-side sub-pumping chamber P1a formed by a diaphragm 8, a waste liquid-side sub-pumping chamber P2a formed by a diaphragm 9, a rod 7, a motor M acting as a driving source, sealing means 12, 13, and a central sealing means 14.

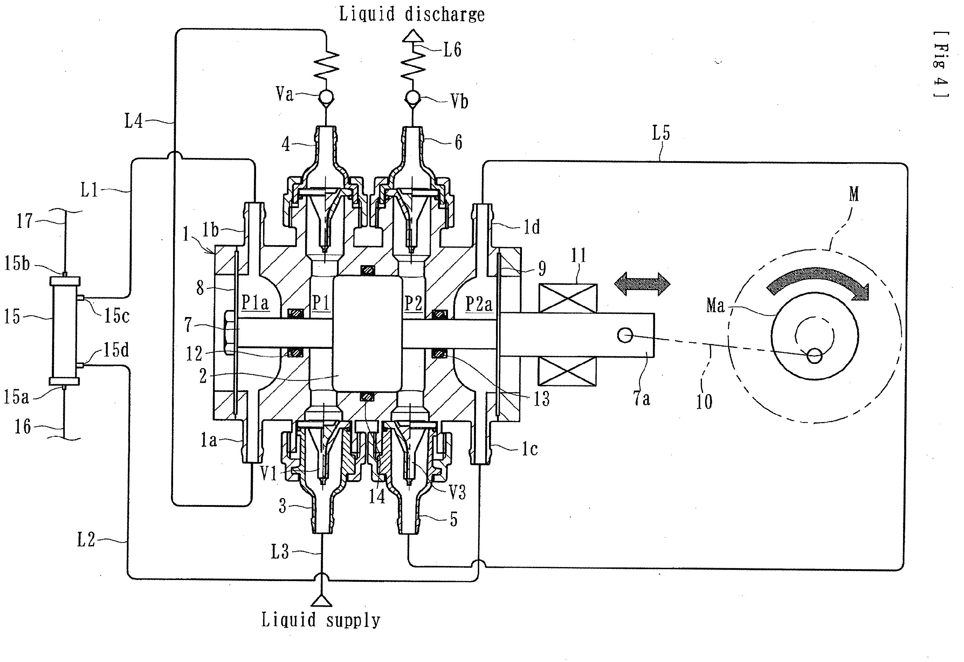

As shown in Fig. 4, the reciprocation pump is connected to the objective apparatus acting as a blood purification apparatus 15. The blood purification apparatus 15 is formed with a blood inlet port 15a, a blood outlet port 15b, dialysate inlet port 15c, and dialysate outlet port 15d. An arterial blood circuit 16 is connected to the blood inlet port 15a and a venous blood circuit 17 is connected to the blood outlet port 15b. In addition, a dialysate supplying line L1 extending from the reciprocation pump is connected to a dialysate inlet port 15c and a dialysate discharging line L2 extending from the reciprocation pump is connected to a dialysate outlet port 15d.

The arterial blood circuit 16 and the venous blood circuit 17 form a blood circuit for extracorporeally circulating a blood of patient and are respectively equipped with an arterial puncture needle and a venous puncture needle (not shown). The blood purification apparatus 15 contains therein an enormous number of hollow fiber membranes (blood purification membranes). The inside of each hollow fiber membrane communicates with the blood inlet port 15a and the blood outlet port 15b and forms a blood flow route. On the other hand, a space between the outer circumferetial surface of each hollow fiber membrane and the inner circumferential surface of the casing of the blood purification apparatus 15 communicates with the dialysate inlet port 15c and the dialysate outlet port 15d to form the dialysate flow route for passing the dialysate (liquid) supplied by the reciprocation pump. Since the hollow fiber membrane is formed with an enormous number of micro pores, unnecessary materials (blood waste materials) can be removed by dialysate via the hollow fiber membranes.

The casing 1 of the reciprocation pump of the present invention is formed of metallic parts or molded hard plastic parts and formed therein the supply liquid-side pumping chamber PI, the waste liquid-side pumping chamber P2, the supply liquid-side sub-pumping chamber P1a, and the waste liquid-side sub-pumping chamber P2a. Projected connection ports 1a, 1b are formed on the casing 1 at the bottom and top of the supply liquid-side sub-pumping chamber P1a and appropriate flow routes (e.g. flexible tubes) can be connected thereto. Similarly, projected connection ports 1c, 1d are formed on the casing 1 at the bottom and top of the waste liquid-side sub-pumping chamber P2a and appropriate flow routes (e.g. flexible tubes) can be connected thereto.

The supply liquid-side pumping chamber P1 is intended to supply the dialysate to the blood purification apparatus 15 (Fig. 4) and a supply liquid-side inlet port 3 and a supply liquid-side outlet port 4 are arranged at the bottom and top of the supply liquid-side pumping chamber P1. Reference characters VI, V2 denote check valves which are mounted respectively on the supply liquid-side inlet port 3 and the supply liquid-side outlet port 4 and act to permit flow of the dialysate from the bottom to the top and to prevent flow of the dialysate from the top to the bottom of the supply liquid-side pumping chamber P1.

The waste liquid-side pumping chamber P2 is intended to discharge a waste liquid to the outside of the reciprocation pump and a waste liquid-side inlet port 5 and a waste liquid-side outlet port 6 are arranged at the bottom and top of the waste liquid-side pumping chamber P2. Reference characters V3, V4 denote check valves which are mounted respectively on the waste liquid-side inlet port 5 and the waste liquid-side outlet port 6 and act to permit flow of the waste liquid from the bottom to the top and to prevent flow of the waste liquid from the top to the bottom of the waste liquid-side pumping chamber P2.

The plunger 2 can be reciprocated between the supply liquid-side pumping chamber P1 and the waste liquid-side pumping chamber P2 by the motor M as a driving source in order to perform the suction and discharge of the dialysate to and from the supply liquid-side pumping chamber P1 and the suction and discharge of the waste dialysate to and from the waste liquid-side pumping chamber P2. That is, the suction of the dialysate from its source and the supply of the dialysate to the blood purification apparatus 15 are repeatingly performed in the supply liquid-side pumping chamber P1 as well as the suction of the waste dialysate from the blood purification apparatus 15 and the discharge of the waste dialysate to the outside of the reciprocation pump are repeatingly performed in the waste liquid-side pumping chamber P2 are simultaneously and repeatingly performed in the waste liquid-side pumping chamber P2 during reciprocal motion of the plunger 2.

The supply liquid-side sub-pumping chamber P1a is arranged adjacent to the supply liquid-side pumping chamber P1 at the external side (left-side) thereof and formed by the diaphragm 8 mounted on the rod 7 and is adapted to perform the suction and discharge of the dialysate via the connection ports 1a, 1b. The diaphragm 8 is secured to the casing 1 with its peripheral edge being fitted into the casing via a sealed manner and adapted to be displaced by the rod 7 to vary the volume of the supply liquid-side sub-pumping chamber P1a.

The waste liquid-side sub-pumping chamber P2a is arranged adjacent to the waste liquid-side pumping chamber P2 at the external side (right-side) thereof and formed by the diaphragm 9 mounted on the rod 7 and is adapted to perform the suction and discharge of the waste dialysate via the connection ports 1c, 1d. The diaphragm 9 is secured to the casing 1 with its peripheral edge being fitted into the casing via a sealed manner and adapted to be displaced by the rod 7 to vary the volume of the waste liquid-side sub-pumping chamber P2a.

The rod 7 extends through the supply liquid-side sub-pumping chamber P1a, the supply liquid-side pumping chamber P1, the waste liquid-side pumping chamber P2 and the waste liquid-side sub-pumping chamber P2a and is connected to the plunger 2 (reciprocation means) and the motor M (driving source) to transmit a driving force of the motor M to the plunger 2 and to reciprocate the plunger 2. More particularly, diaphragms 8, 9 are mounted respectively on the distal end and the proximal end of the rod 7 and the plunger 2 is also mounted on the rod 7 at a substantially central portion between the diaphragms 8, 9.

The supply liquid-side sub-pumping chamber P1a can be communicated with the supply liquid-side pumping chamber P1 so that it permits the dialysate to be flowed therethrough. The diaphragm 8 is interlocked with the plunger 2 and varies the volume of the supply liquid-side sub-pumping chamber P1a reversely to the volume variation of the supply liquid-side pumping chamber P1 (i.e. the volume of the supply liquid-side sub-pumping chamber P1a is reduced in accordance with increase of the volume of the supply liquid-side pumping chamber P1, and on the contrary the volume of the supply liquid-side sub-pumping chamber P1a is increased in accordance with reduction of the volume of the supply liquid-side pumping chamber P1). Similarly, the waste liquid-side sub-pumping chamber P2a can be communicated with the waste liquid-side pumping chamber P2 so that it permits the waste dialysate to be flowed therethrough. The diaphragm 9 is interlocked with the plunger 2 and varies the volume of the waste liquid-side sub-pumping chamber P2a reversely to the volume variation of the waste liquid-side pumping chamber P2 (i.e. the volume of the waste liquid-side sub-pumping chamber P2a is reduced in accordance with increase of the volume of the waste liquid-side pumping chamber P2, and on the contrary the volume of the waste liquid-side sub-pumping chamber P2a is increased in accordance with reduction of the volume of the waste liquid-side pumping chamber P2).

As shown in Fig. 1, a crosshead 7a is mounted on the proximal end of the rod 7 and received through a crosshead cylinder 11 for guiding the reciprocal motion of the rod 7. An output shaft Ma of the motor M is connected to the crosshead 7a via a connecting rod 10 and thus the rod 7 can be reciprocated by the rotation of the motor M via the connecting rod 10.

Sealing means 12, 13 comprise sealing parts in common use such as O-rings arranged respectively in walls through which the rod 7 extends for partitioning the supply liquid-side pumping chamber P1 and the supply liquid-side sub-pumping chamber P1a as well as the waste liquid-side pumping chamber P2 and the waste liquid-side sub-pumping chamber P2a. The sealing means 12, 13 allow the reciprocal sliding motion of the rod 7 and prevent leakage of the liquid between the supply liquid-side pumping chamber P1 and the supply liquid-side sub-pumping chamber P1a as well as the waste liquid-side pumping chamber P2 and the waste liquid-side sub-pumping chamber P2a.

A central sealing means 14 also comprises a sealing part in common use such as an O-ring arranged between the supply liquid-side pumping chamber P1 and the waste liquid-side pumping chamber P2 at a substantially reciprocating center of the plunger 2 to seal the supply liquid-side pumping chamber P1 and the waste liquid-side pumping chamber P2 from each other. That is, the supply liquid-side pumping chamber P1 is sealed by the sealing means 12 and the central sealing means 14 and the waste liquid-side pumping chamber P2 is sealed by the sealing means 13 and the central sealing means 14.

According to the present invention, it is structured that the dialysate is supplied to the blood purification apparatus 15 via both the supply liquid-side pumping chamber P1 and the supply liquid-side sub-pumping chamber P1a and the waste dialysate is discharged from the blood purification apparatus 15 via both the waste liquid-side pumping chamber P2 and the waste liquid-side sub-pumping chamber P2a. More particularly as shown in Fig. 4, the dialysate supplying line L1 is connected between the dialysate inlet port 15c of the blood purification apparatus 15 and the projected connection port 1b and the dialysate discharging line L2 is connected between the dialysate discharging port 15d of the blood purification apparatus 15 and the projected connection port 1c (Such a connection shown in Fig. 4 is hereinafter referred to as "first condition of connection").

In this first connection of connection, a flow route L4 forming part of the dialysate supplying line L1 is connected between the projected connection port 1a and the supply liquid-side outlet port 4 and the supply liquid-side inlet port 3 is connected to a flow route L3 forming part of the dialysate supplying line L1. The flow route L3 is further connected to the supplying source (not shown) of the dialysate conditioned to a predetermined concentration and thus the dialysate fed from the supplying source can be supplied to the blood purification apparatus 15 through the supply liquid-side pumping chamber P1 and the supply liquid-side sub-pumping chamber P1a.

On the other hand, a flow route L5 forming part of the dialysate discharging line L2 is connected to the projected connection port 1d and the waste liquid-side inlet port 5 and the waste liquid-side outlet port 6 is connected to a flow route L6 forming part of the dialysate discharging line L2. The flow route L6 is connected to any discharged liquid recovering means (not shown) and thus the discharged liquid from the blood purification apparatus 15 can be recovered to the discharged liquid recovering means through the waste liquid-side sub-pumping chamber P2a and the waste liquid-side pumping chamber P2. Reference characters Va, Vb in Fig. 4 denotes back pressure valves connected respectively to the flow route L4, L6.

When the plunger 2 is moved by the motor M toward the right in Fig. 4, the dialysate is sucked into the supply liquid-side pumping chamber P1 from the dialysate supplying source and simultaneously the diaphragm 8 is also pulled and thus deflected toward the right. This reduces the volume of the supply liquid-side sub-pumping chamber P1a and thus the dialysate is supplied to the blood purification apparatus 15 from the supply liquid-side sub-pumping chamber P1a via the dialysate supplying line L1. On the other hand, when the plunger 2 is moved by the motor M toward the right in Fig. 4, the dialysate is supplied to the blood purification apparatus 15 from the supply liquid-side pumping chamber P1 and simultaneously the diaphragm 8 is also pushed and thus deflected toward the left. This increases the volume of the supply liquid-side sub-pumping chamber P1a and thus part of the dialysate supplied to the blood purification apparatus 15 is absorbed or supplemented by the supply liquid-side sub-pumping chamber P1a.

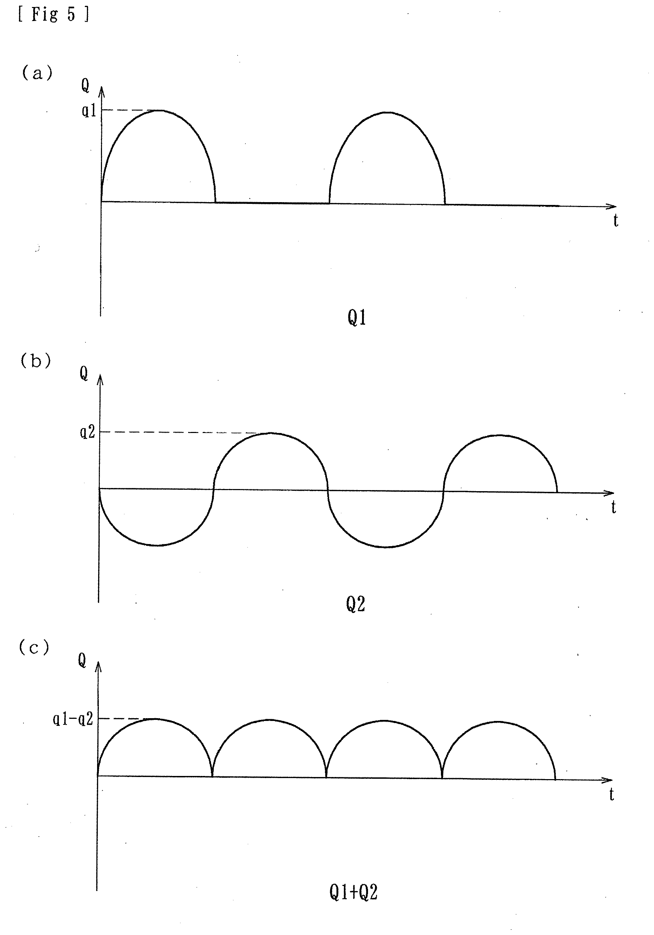

In the first condition of connection, the pulsation (i.e. a variation of flow rate per unit lapse of time) of a discharged flow from the supply liquid-side pumping chamber P1 and the pulsation of a sucked flow into the waste liquid-side pumping chamber P2 have a flow rate waveform illustrated in Fig. 5(a), when the flow rate in the flow route L4 and the flow rate in the flow route L5 are respectively defined as Q1. On the other hand, the pulsation of a discharged flow from the supply liquid-side sub-pumping chamber P1a and a discharged flow from the waste liquid-side sub-pumping chamber P2a have a flow rate waveform shown in Fig. 5(b), when the discharged flow rates from these chambers P1a, P2a are defined as Q2. The actual pulsations in the dialysate supplying line L1 and the dialysate discharging line L2 are determined as a synthesis of these pulsations in Figs. 5(a) and 5(b) and illustrated in Fig. 5(c). As can be understood, the synthesized pulsation (amplitude in Fig.5(c)) is reduced.

Since it is structured so that the reciprocating motion of the plunger 2 enables to supply the dialysite to the blood purification apparatus 15 through the supply liquid-side pumping chamber P1 and the supply liquid-side sub-pumping chamber P1a as well as to discharge the waste dialysate from the blood purification apparatus 15 to the outside through the waste liquid-side sub-pumping chamber P2a and the waste liquid-side pumping chamber P2, it is possible to reduce the pulsation of the dialysate or waste dialysate caused by the reciprocating motion of the plunger 2. Especially under the first condition of connection, it is possible to reduce the pulsation during the supply and discharge of the dialysate to and from the blood purification apparatus 15 and thus to prevent the purification membrane of the blood purification apparatus 15 from being damaged.

According to the present invention, if the discharging volumes of the supply liquid-side pumping chamber P1 and the waste liquid-side pumping chamber P2 are set about twice those of the supply liquid-side sub-pumping chamber P1a and the waste liquid-side sub-pumping chamber P2a, the pulsation caused during the supply of dialysate from the reciprocation pump to the blood purification apparatus 15 can be reduced. In this case the pulsation will be reduced if the discharging volumes of the supply liquid-side pumping chamber P1 and the waste liquid-side pumping chamber P2 are set larger than those of the supply liquid-side sub-pumping chamber P1a and the waste liquid-side sub-pumping chamber P2a.

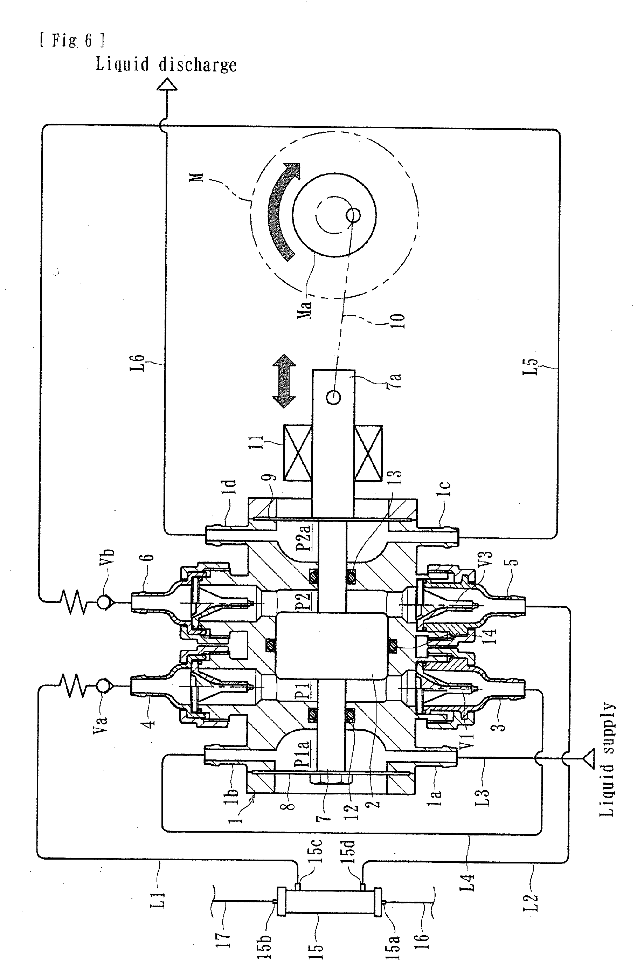

In place of the first condition of connection, it is possible, as shown in Fig. 6, to connect the dialysate supplying line L1 between the dialysate inlet port 15c of the blood purification apparatus 15 and the supply liquid-side outlet port 4 and to connect the dialysate discharging line L2 between the dialysate discharging port 15d of the blood purification apparatus 15 and the waste liquid-side inlet port 5 (Such a connection is hereinafter referred to as "second condition of connection".).

In the second condition of connection, a flow route L4 forming part of the dialysate supplying line L1 is connected between the projected connection port 1b and the supply liquid-side inlet port 3 and the projected connection port 1a is connected to a flow route L3 forming part of the dialysate supplying line L1. The flow route L3 is further connected to the supplying source (not shown) of the dialysate conditioned to a predetermined concentration and thus the dialysate fed from the supplying source can be supplied to the blood purification apparatus 15 through the supply liquid-side sub-pumping chamber P1a and the supply liquid-side pumping chamber P1.

On the other hand, a flow route L5 forming part of the dialysate discharging line L2 is connected to the waste liquid-side outlet port 6 and the projected connection port 1c and the projected connection port 1d is connected to a flow route L6 forming part of the dialysate discharging line L2. The flow route L6 is connected to any discharged liquid recovering means (not shown) and thus the discharged liquid from the blood purification apparatus 15 can be recovered to the discharged liquid recovering means through the waste liquid-side pumping chamber P2 and the waste liquid-side sub-pumping chamber P2a.

In the second condition of connection, the pulsation (i.e. a variation of flow rate per unit lapse of time) has a flow rate waveform illustrated in Fig. 5(a), when the flow rate in the flow route L4 (i.e. flow rate of a sucked flow into the supply liquid-side pumping chamber P1) and the flow rate in the flow route L5 (i.e. flow rate of a discharged flow from the waste liquid-side pumping chamber P2) are respectively defined as Q1. On the other hand, the pulsation has a flow rate waveform shown in Fig. 5(b), when the sucked flow rates into the supply liquid-side sub-pumping chamber P1a and the waste liquid-side sub-pumping chamber P2a are defined as Q2. The pulsation in the flow routes L3, L6 is illustrated in Fig. 5(c) which is a synthesis of these pulsations in Figs. 5(a) and 5(b). As can be understood, the synthesized pulsation is reduced (i.e. the amplitude in Fig.5(c) is reduced as compared with those in Figs. 5(a) and 5(b)).

Since it is structured so that the reciprocating motion of the plunger 2 enables to supply the dialysite to the blood purification apparatus 15 through the supply liquid-side sub-pumping chamber P1a and the supply liquid-side pumping chamber P1 as well as to discharge the waste dialysate from the blood purification apparatus 15 to the outside through the waste liquid-side pumping chamber P2 and the waste liquid-side sub-pumping chamber P2a, it is possible to reduce the pulsation of the dialysate or waste dialysate caused by the reciprocating motion of the plunger 2. Especially under the second condition of connection, it is possible to reduce the pulsation during the supply and discharge of the dialysate to and from the supply liquid-side sub-pumping chamber P1a as well as to reduce the volume of a mixing chamber to be connected to the source of dialysate for obtaining a desirable concentration of dialysate by mixing dense dialysate with water and thus to reduce the size of a blood purification apparatus.

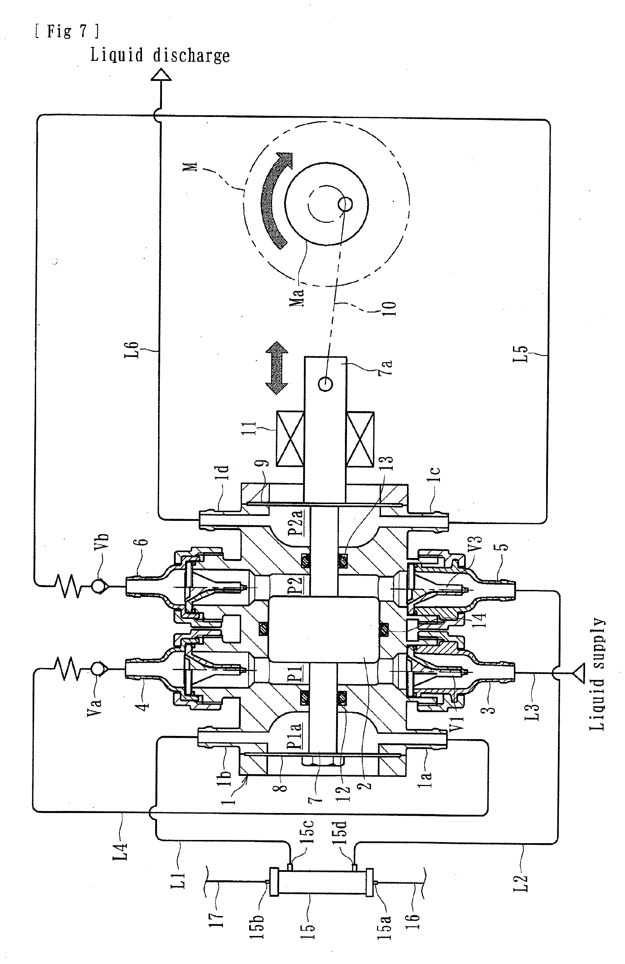

Furthermore, in place of the first and second conditions of connection, it is possible, as shown in Fig. 7, to connect the dialysate supplying line L1 between the dialysate inlet port 15c of the blood purification apparatus 15 and the projected connection port 1b and to connect the dialysate discharging line L2 between the dialysate discharging port 15d of the blood purification apparatus 15 and the waste liquid-side inlet port 5 (Such a connection is hereinafter referred to as "third condition of connection".).

In this third condition of connection, a flow route L4 forming part of the dialysate supplying line L1 is connected between the supply liquid-side outlet port 4 and the projected connection port 1a and the supply liquid-side inlet port 3 is connected to a flow route L3 forming part of the dialysate supplying line L1. The flow route L3 is further connected to the supplying source (not shown) of the dialysate conditioned to a predetermined concentration and thus the dialysate fed from the supplying source can be supplied to the blood purification apparatus 15 through the supply liquid-side pumping chamber P1 and the supply liquid-side sub-pumping chamber P1a.

On the other hand, a flow route L5 forming part of the dialysate discharging line L2 is connected to the waste liquid-side outlet port 6 and the projected connection port 1c and the projected connection port 1d is connected to a flow route L6 forming part of the dialysate discharging line L2. The flow route L6 is connected to any discharged liquid recovering means (not shown) and thus the discharged liquid from the blood purification apparatus 15 can be recovered to the discharged liquid recovering means through the waste liquid-side pumping chamber P2 and the waste liquid-side sub-pumping chamber P2a.

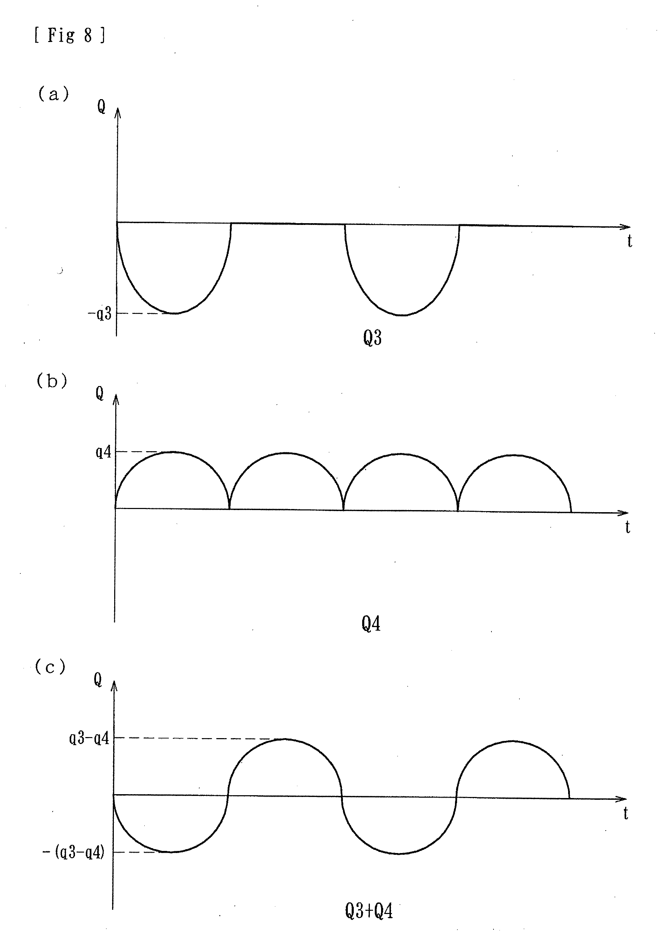

In the third condition of connection, a variation of flow rate per unit lapse of time (i.e. the pulsation) has a flow rate waveform illustrated in Fig. 8(a), when the suction flow rate sucked into the waste liquid-side pumping chamber P2 (i.e. flow rate of the dialysate discharging line L2) is defined as -Q3 (the negative value means a flow rate discharged from the blood purification apparatus 15). On the other hand, the flow rate Q4 (the positive value means a flow rate supplied to the blood purification apparatus 15) of the dialysate supplying line L1 (i.e. synthesis of variation of flow rate in the supply liquid-side pumping chamber P1 and the supply liquid-side sub-pumping chamber) has a flow rate waveform illustrated in Fig. 8(b). The synthesis (Q3 + Q4) of the variation of the flow rate exhibits the pulsation in the blood purification apparatus 15 as shown in Fig. 8(c). It As can be understood, the pulsation is intentionally caused.

In the third condition of connection, the flow rate waveform of Fig. 8(c) can be obtained by differentiate the flow rate waveform from the supply liquid-side sub-pumping chamber P1a and the flow rate waveform into the waste liquid-side pumping chamber P2 (the flow rate waveform from the supply liquid-side pumping chamber P1a is a repeating waveform of small pulsation, on the other hand the flow rate waveform to the waste liquid-side pumping chamber P2 is a repeating waveform of presence and absence of flow), although the flow rate of the dialysate flowing into the blood purification apparatus 15 and the flow rate of the waste dialysate flowing out from the blood purification apparatus 15 are same. The flow rate waveform of Fig. 8(c) enables to positively and intentionally perform the flow-in and flow-out of the dialysate through the blood purification membrane within the blood purification apparatus 15. In this case, it is possible to discharge the dialysate by connecting the dialysate supplying line L1 between the dialysate inlet port 15c and the supply liquid-side outlet port 4 as well as by connecting the dialysate discharging line L2 between the dialysate discharging port 15d and the projected connection port 1c and then by passing the dialysate through the waste liquid-side pumping chamber P2. In this case, the flow rate waveform may be reversed to that in the third condition of connection (i.e. the flow rate waveform from the supply liquid-side pumping chamber P1 is a repeating waveform of presence and absence of flow, on the other hand the flow rate waveform to the waste liquid-side pumping chamber P2 is a repeating waveform of small pulsation).

As shown in the first, second and third conditions of connection, since the order of flow of the dialysate through the supply liquid-side pumping chamber P1 and the supply liquid-side sub-pumping chamber P1a and/or the order of flow of the waste dialysate through the waste liquid-side pumping chamber and the waste liquid-side sub-pumping chamber are arbitrarily selectable to arbitrarily control pulsation caused by the reciprocating motion of the reciprocation means, it is possible to set the pulsation at an arbitral position of the pump as having a desirable flow rate waveform.

That is, the flow-in and flow-out of the dialysate through the blood purification membrane within the blood purification apparatus 15 are repeated when the dialysate is flowed from the supply liquid-side pumping chamber P1 to the supply liquid-side sub-pumping chamber P1a as well as the waste dialysate is flowed from the waste liquid-side sub-pumping chamber P2 to the waste liquid-side sub-pumping chamber P2a. Thus it is possible to reduce the pulsation downstream of the waste liquid-side sub-pumping chamber P2a. On the other hand, the flow-in and flow-out of the dialysate through the blood purification membrane within the blood purification apparatus 15 are repeated when the dialysate is flowed from the supply liquid-side sub-pumping chamber P1a to the supply liquid-side pumping chamber P1 as well as the waste dialysate is flowed from the waste liquid-side sub-pumping chamber P2a to the waste liquid-side pumping chamber P2. Accordingly, it is possible to reduce the pulsation upstream of the supply liquid-side sub pumping chamber P1a. Thus it is possible to set the pulsation at an arbitral position of the pump as having a desirable flow rate waveform, since the pulsation caused by the reciprocating motion of the plunger 2 can be arbitrarily controlled by arbitrarily selecting the condition of connection.

According to the present invention, since the reciprocation pump is provided with the supply liquid-side sub-pumping chamber P1a and the waste liquid-side sub-pumping chamber P2a in addition to the supply liquid-side pumping chamber P1 and the waste liquid-side pumping chamber P2 and structured so that the reciprocating motion of the reciprocation means enables the liquid to be supplied to the blood purification apparatus 15 through the supply liquid-side pumping chamber P1 and the supply liquid-side sub-pumping chamber P1a as well as enables the waste liquid from the blood purification apparatus 15 to be discharged to the outside through the waste liquid-side pumping chamber P2 and the waste liquid-side sub-pumping chamber P2a, it is possible to reduce the pulsation of the liquid and waste liquid caused by the reciprocating motion of the reciprocation means.

In addition, since the reciprocation pump further comprises a rod 7 extending through the supply liquid-side sub-pumping chamber P1a, the supply liquid-side pumping chamber P1, the waste liquid-side pumping chamber P2 and the waste liquid-side sub-pumping chamber P2a and being connected to the plunger 2 and the motor M to transmit a driving force of the motor M to the plunger 2; and diaphragms 8, 9 respectively mounted on the distal end and the proximal end of the rod 7 to form the supply liquid-side sub-pumping chamber P1a and the waste liquid-side sub-pumping chamber P2a respectively, it is possible to construct the supply liquid-side sub-pumping chamber P1a interlocked with the plunger 2 to vary its volume reversely to that of the volume variation of the supply liquid-side pumping chamber P1 and the waste liquid-side sub-pumping chamber P2a interlocked with the plunger 2 to vary its volume reversely to that of the volume variation of the waste liquid-side pumping chamber P2. In addition, it enables to eliminate any separate sealing means such as shaft seals at mounting portions of the diaphragms 8, 9.

Furthermore since the reciprocation means comprises a plunger 2 formed on the rod 7 to form the supply liquid-side pumping chamber P1 and the waste liquid-side pumping chamber P2, it is possible to use a conventional plunger and thus to reduce the manufacturing cost. In addition, since the supply liquid-side sub-pumping chamber P1a and the waste liquid-side sub-pumping chamber P2a are arranged respectively adjacent to the supply liquid-side pumping chamber P1 and the waste liquid-side pumping chamber P2 at the external side thereof; and sealing means 12, 13 are arranged respectively in walls through which the rod 7 extends for partitioning the supply liquid-side pumping chamber P1 and the supply liquid-side sub-pumping chamber P1a as well as the waste liquid-side pumping chamber P2 and the waste liquid-side sub-pumping chamber P2a, it is possible to prevent the dialysate to be supplied to the blood purification apparatus 15 as well as the waste dialysate from the blood purification apparatus 15 from being leaked to the outside of the reciprocation pump.

In addition, since a central sealing means 14 is arranged between the supply liquid-side pumping chamber P1 and the waste liquid-side pumping chamber P2 at a substantially reciprocating center of the plunger 2 to seal the supply liquid-side pumping chamber P1 and the waste liquid-side pumping chamber P2 from each other, the leaked dialysate can flow from the supply liquid-side pumping chamber P1 to the waste liquid-side pumping chamber P2 or the leaked waste dialysate can flow from the waste liquid-side pumping chamber P2 to the supply liquid-side pumping chamber P1, and thus it is possible to prevent the leaked dialysate and waste dialysate from being leaked to the outside of the reciprocation pump.

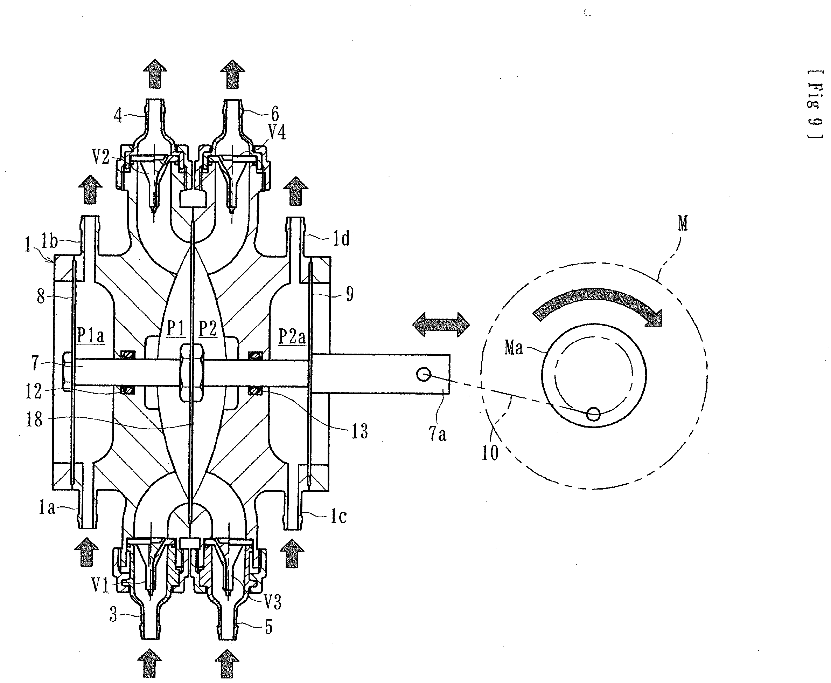

Although the preferable embodiment of the present invention has been described above, the present invention is not limited to this. Fig. 9 shows another embodiment of the present invention which comprises a diaphragm 18 in place of the plunger 2 mounted on the rod 7 for forming the supply liquid-side pumping chamber P1 and the waste liquid side pumping chamber P2. Also in this embodiment, since the supply liquid-side sub-pumping chamber P1a and the waste liquid-side sub-pumping chamber P2a are arranged respectively adjacent to the supply liquid-side pumping chamber P1 and the waste liquid-side pumping chamber P2 at the external side thereof; and sealing means 12, 13 are arranged respectively in walls through which the rod 7 extends for partitioning the supply liquid-side pumping chamber P1 and the supply liquid-side sub-pumping chamber P1a as well as the waste liquid-side pumping chamber P2 and the waste liquid-side sub-pumping chamberP2a, it is possible to prevent the dialysate to be supplied to the blood purification apparatus 15 as well as the waste dialysate from the blood purification apparatus 15 from being leaked to the outside of the reciprocation pump. Furthermore in this embodiment, since the reciprocation means is formed by the diaphragm 18, it is possible to eliminate the central sealing means 14 for sealingly form the supply liquid-side pumping chamber P1 and the waste liquid-side pumping chamber P2 as compared with a case using plunger 2 for this purpose.

Furthermore, the present invention is not limited to a reciprocation pump used for supplying dialysate to the blood purification apparatus 15 and can be applied to other reciprocation means (whole industrial pumps used for common use) which supply liquids to other objective apparatus than the blood purification apparatus. Although in such a case, it is possible to reduce the pulsation of the liquid and waste liquid caused by the reciprocating motion of the reciprocation means by providing the reciprocation pump with the supply liquid-side sub-pumping chamber and the waste liquid-side sub-pumping chamber in addition to the supply liquid-side pumping chamber and the waste liquid-side pumping chamber and by structuring so that the reciprocating motion of the reciprocation means enables the liquid to be supplied to the objective apparatus through the supply liquid-side pumping chamber and the supply liquid-side sub-pumping chamber as well as enables the waste liquid from the objective apparatus to be discharged to the outside through the waste liquid-side pumping chamber and the waste liquid-side sub-pumping chamber.

### Applicability in Industry

The present invention can be applied to a reciprocation pump and a dialysis apparatus equipped with such a reciprocation pump having a different external appearance from that shown in the accompanied drawings or having additional functions, if the reciprocation pump comprises a supply liquid-side sub-pumping chamber for containing and sending out liquid adapted to be communicated with the supply liquid-side pumping chamber and interlocked with the reciprocation means to vary its volume reversely to that of the volume variation of the supply liquid-side pumping chamber; and a waste liquid-side sub-pumping chamber for containing and sending out liquid adapted to be communicated with the waste liquid-side pumping chamber and interlocked with the reciprocation means to vary its volume reversely to that of the volume variation of the waste liquid-side pumping chamber; and the reciprocating motion of the reciprocation means enables the liquid to be supplied to the objective apparatus through the supply liquid-side pumping chamber and the supply liquid-side sub-pumping chamber as well as enables the waste liquid from the objective apparatus to be discharged to the outside through the waste liquid-side pumping chamber and the waste liquid-side sub-pumping chamber.

### Description of Reference Numerals

- 1: casing
- 2: plunger (reciprocation means)
- 3: supply liquid-side inlet port
- 4: supply liquid-side outlet port
- 5: waste liquid-side inlet port
- 6: waste liquid-side outlet port
- 7: rod
- 8: diaphragm
- 9: diaphragm
- 10: connecting rod
- 11: crosshead cylinder
- 12: sealing means
- 13: sealing means
- 14: central sealing means
- 15: blood purification apparatus (objective apparatus)
- 16: arterial-side blood circuit
- 17: venous-side blood circuit
- 18: diaphragm (reciprocation means)
- M: motor (driving source)
- P1: supply liquid-side pumping chamber
- P1a: supply liquid-side sub-pumping chamber
- P2: waste liquid-side pumping chamber
- P2a: waste liquid-side sub-pumping chamber

## Claims

1. A system comprising an objective apparatus and a reciprocation pump, the reciprocation pump comprising:
a supply liquid-side pumping chamber (P1) configured to supply a liquid from a source to the objective apparatus (15);
a waste liquid-side pumping chamber (P2) configured to discharge a waste liquid discharged from the objective apparatus (15) to the outside;
a reciprocation means (2) being able to reciprocate between the supply liquid-side pumping chamber (P1) and the waste liquid-side pumping chamber (P2), the reciprocating motion of the reciprocation means (2) performing suction and discharge of the liquid to and from the supply liquid-side pumping chamber (P1) as well as suction and discharge of the waste liquid to and from the waste liquid-side pumping chamber (P2); and
a driving source (M) for driving the reciprocation means (2);
the reciprocation pump adapted to supply the liquid from the source to the objective apparatus (15) with the supply liquid-side pumping chamber (P1) and to discharge the waste liquid from the objective apparatus (15) with the waste liquid-side pumping chamber (P2) to the outside;
the reciprocation pump further comprises a supply liquid-side sub-pumping chamber (P1a) for containing and sending out liquid adapted to be communicated with the supply liquid-side pumping chamber (P1) and interlocked with the reciprocation means (2) to vary its volume reversely to that of the volume variation of the supply liquid-side pumping chamber (P1); and
a waste liquid-side sub-pumping chamber (P2a) for containing and sending out liquid adapted to be communicated with the waste liquid-side pumping chamber (P2) and interlocked with the reciprocation means (2) to vary its volume reversely to that of the volume variation of the waste liquid-side pumping chamber (P2); and
that the reciprocating motion of the reciprocation means (2) enables the liquid to be supplied to the objective apparatus (15) through the supply liquid-side pumping chamber (P1) and the supply liquid-side sub-pumping chamber (P1a) as well as enables the waste liquid from the objective apparatus (15) to be discharged to the outside through the waste liquid-side pumping chamber (P2) and the waste liquid-side sub-pumping chamber (P2a);
wherein the supply liquid-side pumping chamber (P1) comprises a supply liquid-side inlet port (3) and a supply liquid-side outlet port (4) and the supply liquid-side sub-pumping chamber (P1a) comprises connection ports (1a and 1b), and
the waste liquid-side pumping chamber (P2) comprises a waste liquid-side inlet port (5) and a waste liquid-side outlet port (6) and the waste liquid-side sub-pumping chamber (P2a) comprises connection ports (1c and 1d).

2. A system of claim 1 wherein the reciprocation pump further comprises:
a rod (7) extending through the supply liquid-side sub-pumping chamber (P1a), the supply liquidside pumping chamber (P1), the waste liquid-side pumping chamber (P2) and the waste liquid-side sub-pumping chamber (P2a) and being connected to the reciprocation means (2) and the driving source (M) to transmit a driving force of the driving source (M) to the reciprocation means (2); and
diaphragms (8, 9) respectively mounted on the distal end and the proximal end of the rod (7) to form the supply liquid-side sub-pumping chamber (P1a) and the waste liquid-side sub-pumping chamber (P2a) respectively.

3. A system of claim 1 or 2 wherein the reciprocating pump is configured to supply liquid from the source to the objective apparatus through the supply liquid-side pumping chamber (P1) via the supply liquid-side sub-pumping chamber (P1a) and to discharge liquid from the objective apparatus to the outside through the waste liquid-side sub-pumping chamber (P2a) via the waste liquid-side pumping chamber (P2).

4. A system of claim 1 or 2 wherein the reciprocating pump is configured to supply liquid from the source to the objective apparatus through the supply liquid-side sub-pumping chamber (P1a) via the supply liquid-side pumping chamber (P1) and to discharge liquid from the objective apparatus to the outside through the waste liquid-side pumping chamber (P2) via the waste liquid-side sub-pumping chamber (P2a).

5. A system of claim 1 or 2 wherein the reciprocating pump is configured to supply liquid from the source to the objective apparatus through the supply liquid-side pumping chamber (P1) via the supply liquid-side sub-pumping chamber (P1a) and to discharge liquid from the objective apparatus to the outside through the waste liquid-side pumping chamber (P2) via the waste liquid-side sub-pumping chamber (P2a).

6. A system of any one of claims 2 to 5 wherein the reciprocation means (2) comprises a plunger (2) formed on the rod (7) to form the supply liquid-side pumping chamber (P1) and the waste liquid-side pumping chamber (P2).

7. A system of any one of claims 2 to 5 wherein the reciprocation means (2) comprises a diaphragm (18) mounted on the rod (7) to form the supply liquid-side pumping chamber (P1) and the waste liquid-side pumping chamber (P2).

8. A system of any one of claims 1 to 7 wherein the objective apparatus (15) is a blood purification apparatus and the liquid is dialysate.

9. A dialysis apparatus equipped with the system of claim 8.

## Patentansprüche

1. System mit einer Zielvorrichtung und einer Hubkolbenpumpe, wobei die Hubkolbenpumpe umfasst:
eine versorgungsflüssigkeitsseitige Pumpenkammer (P1), die so konfiguriert ist, dass sie eine Flüssigkeit von einer Quelle zu der Zielvorrichtung (15) liefert;
eine abfallflüssigkeitsseitige Pumpenkammer (P2), die so konfiguriert ist, dass sie eine von der Zielvorrichtung (15) abgegebene Abfallflüssigkeit nach außen abgibt;
eine Reziprokiereinrichtung (2), die in der Lage ist, sich zwischen der versorgungsflüssigkeitsseitigen Pumpenkammer (P1) und der abfallflüssigkeitsseitigen Pumpenkammer (P2) hin- und herzubewegen, wobei die Hin- und Herbewegung der Reziprokiereinrichtung (2) das Ansaugen und Ausstoßen der Flüssigkeit zu und aus der versorgungsflüssigkeitsseitigen Pumpenkammer (P1) sowie das Ansaugen und Ausstoßen der Abfallflüssigkeit zu und aus der abfallflüssigkeitsseitigen Pumpenkammer (P2) durchführt; und eine Antriebsquelle (M) für den Antrieb der Reziprokiereinrichtung (2);
die Hubkolbenpumpe, die geeignet ist, die Flüssigkeit von der Quelle zu der Zielvorrichtung (15) mit der versorgungsflüssigkeitsseitigen Pumpenkammer (P1) zu liefern und die Abfallflüssigkeit von der Zielvorrichtung (15) mit der abfallflüssigkeitsseitigen Pumpenkammer (P2) nach außen abzugeben;
wobei die Hubkolbenpumpe ferner eine versorgungsflüssigkeitsseitige Nebenpumpenkammer (P1a) zum Aufnehmen und Abgeben von Flüssigkeit umfasst, die mit der versorgungsflüssigkeitsseitigen Pumpenkammer (P1) in Verbindung gebracht werden kann und mit der Reziprokiereinrichtung (2) verriegelt ist, um ihr Volumen umgekehrt zu dem der Volumenänderung der versorgungsflüssigkeitsseitigen Pumpenkammer (P1) zu verändern; und eine abfallflüssigkeitsseitige Nebenpumpenkammer (P2a) zum Aufnehmen und Abgeben von Flüssigkeit, die mit der abfallflüssigkeitsseitigen Pumpenkammer (P2) in Verbindung gebracht werden kann und mit der Reziprokiereinrichtung (2) verriegelt ist, um ihr Volumen umgekehrt zu dem der Volumenänderung der abfallflüssigkeitsseitigen Pumpenkammer (P2) zu verändern; und
dass die Hin- und Herbewegung der Reziprokiereinrichtung (2) es ermöglicht, die Flüssigkeit der Zielvorrichtung (15) durch die versorgungsflüssigkeitsseitige Pumpenkammer (P1) und die versorgungsflüssigkeitsseitige Nebenpumpenkammer (P1a) zuzuführen sowie die Abfallflüssigkeit von der Zielvorrichtung (15) durch die abfallflüssigkeitsseitige Pumpenkammer (P2) und die abfallflüssigkeitsseitige Nebenpumpenkammer (P2a) nach außen abzuführen;
wobei die versorgungsflüssigkeitsseitige Pumpenkammer (P1) eine versorgungsflüssigkeitsseitige Einlassöffnung (3) und eine versorgungsflüssigkeitsseitige Auslassöffnung (4) umfasst und die versorgungsflüssigkeitsseitige Nebenpumpenkammer (P1a) Verbindungsöffnungen (1a und 1b) umfasst, und
die abfallflüssigkeitsseitige Pumpenkammer (P2) eine abfallflüssigkeitsseitige Einlassöffnung (5) und eine abfallflüssigkeitsseitige Auslassöffnung (6) umfasst und die abfallflüssigkeitsseitige Nebenpumpenkammer (P2a) Verbindungsöffnungen (1c und 1d) umfasst.

2. System nach Anspruch 1, wobei die Hubkolbenpumpe ferner umfasst:
eine Stange (7), die sich durch die versorgungsflüssigkeitsseitige Nebenpumpenkammer (P1a), die versorgungsflüssigkeitsseitige Pumpenkammer (P1), die abfallflüssigkeitsseitige Pumpenkammer (P2) und die abfallflüssigkeitsseitige Nebenpumpenkammer (P2a) erstreckt und mit der Reziprokiereinrichtung (2) und der Antriebsquelle (M) verbunden ist, um eine Antriebskraft der Antriebsquelle (M) auf die Reziprokiereinrichtung (2) zu übertragen; und
Membranen (8, 9), die jeweils am distalen Ende und am proximalen Ende der Stange (7) angebracht sind, um die versorgungsflüssigkeitsseitige Nebenpumpenkammer (P1a) bzw. die abfallflüssigkeitsseitige Nebenpumpenkammer (P2a) zu bilden.

3. System nach Anspruch 1 oder 2, bei dem die Hubkolbenpumpe so konfiguriert ist, dass sie Flüssigkeit von der Quelle durch die versorgungsflüssigkeitsseitige Pumpenkammer (P1) über die versorgungsflüssigkeitsseitige Nebenpumpenkammer (P1a) der Zielvorrichtung zuführt und Flüssigkeit von der Zielvorrichtung durch die abfallflüssigkeitsseitige Nebenpumpenkammer (P2a) über die abfallflüssigkeitsseitige Pumpenkammer (P2) nach außen abgibt.

4. System nach Anspruch 1 oder 2, wobei die Hubkolbenpumpe so konfiguriert ist, dass sie Flüssigkeit von der Quelle durch die versorgungsflüssigkeitsseitige Nebenpumpenkammer (P1a) über die versorgungsflüssigkeitsseitige Pumpenkammer (P1) der Zielvorrichtung zuführt und Flüssigkeit von der Zielvorrichtung durch die abfallflüssigkeitsseitige Pumpenkammer (P2) über die abfallflüssigkeitsseitige Nebenpumpenkammer (P2a) nach außen abgibt.

5. System nach Anspruch 1 oder 2, wobei die Hubkolbenpumpe so konfiguriert ist, dass sie Flüssigkeit von der Quelle zu der Zielvorrichtung durch die versorgungsflüssigkeitsseitige Pumpenkammer (P1) über die versorgungsflüssigkeitsseitige Nebenpumpenkammer (P1a) zuführt und Flüssigkeit von der Zielvorrichtung durch die abfallflüssigkeitsseitige Pumpenkammer (P2) über die abfallflüssigkeitsseitige Nebenpumpenkammer (P2a) nach außen abgibt.

6. System nach einem der Ansprüche 2 bis 5, wobei die Reziprokiereinrichtung (2) einen auf der Stange (7) ausgebildeten Kolben (2) umfasst, um die versorgungsflüssigkeitsseitige Pumpenkammer (P1) und die abfallflüssigkeitsseitige Pumpenkammer (P2) zu bilden.

7. System nach einem der Ansprüche 2 bis 5, wobei die Hin- und Herbewegungseinrichtung (2) eine auf der Stange (7) angebrachte Membran (18) aufweist, um die versorgungsflüssigkeitsseitige Pumpenkammer (P1) und die abfallflüssigkeitsseitige Pumpenkammer (P2) zu bilden.

8. System nach einem der Ansprüche 1 bis 7, wobei die Zielvorrichtung (15) eine Blutreinigungsvorrichtung ist und die Flüssigkeit Dialysat ist.

9. Ein Dialysegerät, das mit dem System nach Anspruch 8 ausgestattet ist.

## Revendications

1. Système comprenant un dispositif d'objectif et une pompe alternative, ladite pompe alternative comprenant :
une chambre de pompe (P1) côté liquide d'alimentation configurée pour effectuer une alimentation d'un liquide depuis une source audit dispositif d'objectif (15) ;
une chambre de pompe (P2) côté liquide usé configurée pour effectuer un déchargement d'un liquide usé qui est déchargé dudit dispositif d'objectif (15) vers l'extérieur ;
des moyens alternatifs (2) apte à effectuer un mouvement de va-et-vient entre ladite chambre de pompe (P1) côté liquide d'alimentation et ladite chambre de pompe (P2) côté liquide usé, le mouvement de va-et-vient des moyens alternatifs (2) effectuant une aspiration et un refoulement de liquide vers ladite chambre de pompe (P1) côté liquide d'alimentation et depuis celle-ci aussi qu'une aspiration et un refoulement de liquide usé vers ladite chambre de pompe (P2) côté liquide usé et depuis celle-ci ; et
une source d'entraînement (M) destinée à entraîner lesdits moyens alternatifs (2) ;
ladite pompe alternative étant adaptée pour effectuer une alimentation de liquide depuis ladite source audit dispositif d'objectif (15) moyennant ladite chambre de pompe (P1) côté liquide d'alimentation et pour effectuer un déchargement de liquide usé depuis ledit dispositif d'objectif (15) vers l'extérieur moyennant ladite chambre de pompe (P2) côté liquide usé ;
ladite pompe alternative comprenant en outre une sous-chambre de pompe (P1a) côté liquide d'alimentation qui est destinée à contenir et envoyer de liquide et qui est apte à être mis en communication avec ladite chambre de pompe (P1) côté liquide d'alimentation, et interverrouillé avec lesdits moyens alternatifs (2) afin de varier son volume de manière inversé à une variation du volume de ladite chambre de pompe (P1) côté liquide d'alimentation ; et
une sous-chambre de pompe (P2a) côté liquide usé qui est destinée à contenir et envoyer de liquide et qui est apte à être mis en communication avec ladite chambre de pompe (P2) côté liquide usé, et interverrouillé avec lesdits moyens alternatifs (2) afin de varier son volume de manière inversé à une variation du volume de ladite chambre de pompe (P2) côté liquide usé ; et
le mouvement de va-et-vient des moyens alternatifs (2) permettant l'alimentation en liquide dudit dispositif d'objectif (15) par ladite chambre de pompe (P1) côté liquide d'alimentation et ladite sous-chambre de pompe (P1a) côté liquide d'alimentation ainsi que le déchargement de liquide usé depuis ledit dispositif d'objectif (15) vers l'extérieur par ladite chambre de pompe (P2) côté liquide usé et ladite sous-chambre de pompe (P2a) côté liquide usé ;
ladite chambre de pompe (P1) côté liquide d'alimentation comprenant un raccord d'entrée (3) côté liquide d'alimentation et un raccord de sortie (4) côté liquide d'alimentation et ladite sous-chambre de pompe (P1a) côté liquide d'alimentation comprenant des raccords de connexion (1a et 1b), et
ladite chambre de pompe (P2) côté liquide usé comprenant un raccord d'entrée (5) côté liquide usé et un raccord de sortie (6) côté liquide usé et ladite sous-chambre de pompe (P2a) côté liquide usé comprenant des raccords de connexion (1c et 1d).

2. Système selon la revendication 1, dans lequel ladite pompe alternative comprend en outre :
une tige (7) qui s'étend à travers ladite sous-chambre de pompe (P1a) côté liquide d'alimentation, ladite chambre de pompe (P1) côté liquide d'alimentation, ladite chambre de pompe (P2) côté liquide usé, et ladite sous-chambre de pompe (P2a) côté liquide usé et qui est relié avec lesdits moyens alternatifs (2) et ladite source d'entraînement (M) pour transmettre une force d'entraînement de ladite source d'entraînement (M) auxdits moyens alternatifs (2) ; et
diaphragmes (8, 9) montés, respectivement, sur l'extrémité distale et l'extrémité proximale de ladite tige (7) pour former, respectivement, ladite sous-chambre de pompe (P1a) côté liquide d'alimentation et ladite sous-chambre de pompe (P2a) côté liquide usé.

3. Système selon la revendication 1 ou 2, dans lequel ladite pompe alternative est configurée pour effectuer une alimentation de liquide depuis la source audit dispositif d'objectif par ladite chambre de pompe (P1) côté liquide d'alimentation via ladite sous-chambre de pompe (P1a) côté liquide d'alimentation et pour effectuer un déchargement de liquide depuis ledit dispositif d'objectif vers l'extérieur par ladite sous-chambre de pompe (P2a) côté liquide usé via ladite chambre de pompe (P2) côté liquide usé.

4. Système selon la revendication 1 ou 2, dans lequel ladite pompe alternative est configurée pour effectuer une alimentation de liquide depuis la source audit dispositif d'objectif par ladite sous-chambre de pompe (P1a) côté liquide d'alimentation via ladite chambre de pompe (P1) côté liquide d'alimentation et pour effectuer un déchargement de liquide depuis ledit dispositif d'objectif vers l'extérieur par ladite chambre de pompe (P2) côté liquide usé via ladite sous-chambre de pompe (P2a) côté liquide usé.

5. Système selon la revendication 1 ou 2, dans lequel ladite pompe alternative est configurée pour effectuer une alimentation de liquide depuis la source audit dispositif d'objectif par ladite chambre de pompe (P1) côté liquide d'alimentation via ladite sous-chambre de pompe (P1a) côté liquide d'alimentation et pour effectuer un déchargement de liquide depuis ledit dispositif d'objectif vers l'extérieur par ladite chambre de pompe (P2) côté liquide usé via ladite sous-chambre de pompe (P2a) côté liquide usé.

6. Système selon l'une quelconque des revendications 2 à 5, dans lequel lesdits moyens alternatifs (2) comprennent un piston plongeur (2) formé sur la tige (7) pour former ladite chambre de pompe (P1) côté liquide d'alimentation et ladite chambre de pompe (P2) côté liquide usé.

7. Système selon l'une quelconque des revendications 2 à 5, dans lequel lesdits moyens alternatifs (2) comprennent un diaphragme (18) monté sur la tige (7) pour former ladite chambre de pompe (P1) côté liquide d'alimentation et ladite chambre de pompe (P2) côté liquide usé.

8. Système selon l'une quelconque des revendications 1 à 7, dans lequel ledit dispositif d'objectif (15) est un appareil de purification de sang et le liquide est un dialysat.

9. Appareil de dialyse équipé avec le système selon la revendication 8.
